**Europäisches Patentamt**

**European Patent Office**

**Office européen des brevets**

(19)

(11) Veröffentlichungsnummer : **0 271 545 B1**

# EUROPÄISCHE PATENTSCHRIFT

(12)

(45) Veröffentlichungstag der Patentschrift :
**21.11.91 Patentblatt 91/47**

(51) Int. Cl.⁵ : **A61B 1/00, A61B 1/30**

(21) Anmeldenummer : **87904027.7**

(22) Anmeldetag : **25.06.87**

(86) Internationale Anmeldenummer :
**PCT/DE87/00287**

(87) Internationale Veröffentlichungsnummer :
**WO 88/00020 14.01.88 Gazette 88/02**

(54) **SEITENBLICK-ENDOSKOP.**

(30) Priorität : **27.06.86 DE 3621509**

(43) Veröffentlichungstag der Anmeldung :
**22.06.88 Patentblatt 88/25**

(45) Bekanntmachung des Hinweises auf die
Patenterteilung :
**21.11.91 Patentblatt 91/47**

(84) Benannte Vertragsstaaten :
**FR GB**

(56) Entgegenhaltungen :
**DE-C- 480 520**
**FR-A- 711 949**
**US-A- 1 901 731**

(73) Patentinhaber : **Richard Wolf GmbH**
**Pforzheimer Strasse 32**
**W-7134 Knittlingen (DE)**

(72) Erfinder : **THON, Hans, Joachim**
**Lindenweg 26**
**W-5305 Alfter-Oedekoven (DE)**

(74) Vertreter : **Wilcken, Hugo, Dr. et al**
**Patentanwälte Dr. Hugo Wilcken Dipl.-Ing.**
**Thomas Wilcken Musterbahn 1**
**W-2400 Lübeck (DE)**

EP 0 271 545 B1

## Beschreibung

Die Erfindung betrifft ein Seitenblick-Endoskop mit einem Instrumentierkanal, der im Arbeitsbereich (Distalende) eine Austrittsöffnung bildet, und mit einem Ablenkelement sowie einer Betätigungseinrichtung für dieses, wobei der Instrumentierkanal am Austrittsende einen gekrümmten Abschnitt aufweist, der im Arbeitsbereich zu der in der seitlichen Wandung des Endoskopes vorgesehenen Austrittsöffnung hin gerichtet ist.

Seitenblick-Endoskope dienen endoskopischen Untersuchungen insbesondere des Zwölffingerdarmes von Lebewesen, insbesondere zur Darstellung der Papilla Vateri (Ausführungsöffnung von Gallen- und Pankreasausgang) mit der Möglichkeit, Instrumente (Katheter, Bürsten, Gewebszangen, Schlingen) in den Bauchspeicheldrüsengang sowie in den Gallengang einzubringen, um so diagnostische bzw. therapeutische Maßnahmen durchzuführen oder auch zur Implantation von Endoprothesen.

Ein Seitenblick-Endoskop der einleitend angeführten Art ist in der FR-A-711 949 beschrieben. Bei diesem Endoskop ist der Arbeitsbereich so beschaffen, daß der im starren Endoskopschaft geführte Instrumentenkanal in Richtung der Längsachse des Endoskopes in einem kleinen Schacht austritt, der sich selbst in einem abgewinkelten Endabschnitt des distalen Endoskopendes befindet. Durch ein in diesem Schacht angelenktes gerades Ablenkelement, das vom Kopfende des Endoskopes mechanisch bewegt werden kann, kann der Austrittswinkel des durch den Instrumentierkanal geführten Instrumentariums verändert werden. Bei einem vollkommen entspannten Ablenkelement tritt das Instrumentarium (Katheter, Führungsdraht, Verweilkatheter) in einem Winkel von etwa 75° zur Längsachse des Endoskopes distalwärts aus. Bei vollständiger Abwinkelung des Ablenkelementes kann ein Austrittswinkel von über 90° zur Längsachse des Endoskopes erreicht werden.

Insbesondere bei kompletter Abwinkelung kommt es infolge konstruktiv bedingter Umstände des Ablenkelementes nicht zu einer Führung des Instrumentariums im Sinne einer Biegungslinie, sondern vielmehr zu einer Knickbildung in Höhe der Ausmündungsstelle des Instrumentierkanals in dem Arbeitsschacht. Hierdurch treten starke Reibungskräfte sowohl durch die Abknickung an der Austrittsöffnung des Instrumentierkanals als auch an der Innenfläche des Abwinkelungshebels auf. In Abhängigkeit von der Kaliberstärke des Instrumentariums und dessen eigensteifigkeit ist dann ein weiteres Vorschieben erheblich erschwert bzw. unmöglich. Insbesondere bei der Verwendung steifer Instrumentarien, wie großkalibrige Verweilkatheter, kann es bei Ablenkung mit dem Ablenkelement infolge der Steifigkeit des Materials zum Auftreten von Scherkräften bzw. Tangentialkräften kommen, die keine achsengerechte Kraftübertragung zulassen und somit keine optimale Lösung für die Einbringung insbesondere von steifen Instrumentarien darstellen.

Die Erfindung liegt daher die Aufgabe zugrunde, ein Seitenblick-Endoskop der eingangs genannten Art dahingehend zu verbessern, daß eine achsengerechte Kraftübertragung möglich ist und somit eine optimale Lösung für die Einbringung insbesondere steifer großkalibriger Instrumentarien geschaffen ist.

Diese Aufgabe wird erfindungsgemäß durch die Merkmale des Patentanspruchs 1 gelöst.

Mit der erfindungsgmeäßen Lösung wird ein Seitenblick-Endoskop geschaffen, das auch bei großkalibrigen und steifen Instrumentarien, wie beispielsweise großkalibrigen Verweilkathetern, das Auftreten von Scher- bzw. Tangentialkräften vermeidet, so daß eine achsengerechte Kraftübertragung möglich ist. Da Scherkräfte weitestgehend beseitigt sind, können Dislokationen des Instrumentariums nach distalwärts, die bei den bekannten Geräten zu einer Unterbrechung der Einbringung führten, praktisch nicht mehr auftreten. Insbesondere wird mit der Erfindung bei korrekter Lage des Distalendes in der Mitte des absteigenden Schenkels des Zwölffingerdarmes die Einführung von Instrumentarien (wie z.B. Führungsdraht oder Verweilkatheter) in den Gallengang auf relativ einfache Weise ermöglicht, wozu eine sichere Ablenkung im Bereich von etwa 90 bis 120°, bezogen auf die Längsachse des Endoskopes, erforderlich ist.

Das Ablenkelement ist im Bereich der Krümmung gelagert und mittels der Betätigungseinrichtung in eine Stellung bewegbar, in der es angrenzend an den Krümmungsbereich seitlich aus dem Endoskop hervorsteht. Um bei korrekter Lage des Distalendes des Seitenblick-Endoskopes in der Mitte des absteigenden Schenkels des Zwölffingerdarmes das Instrumentarium (z.B. Führungsdraht oder Verweilkatheter) in den Gallengang einzubringen, muß eine Ablenkung des Instrumentariums mit einem Winkel zwischen 90 und 120°, bezogen auf die Längsachse des Endoskopes, erreicht werden, um eine der Lage des Gallenganges entsprechende achsengerechte Schubkraftübertragung erreichen zu können. Diese Voraussetzung ist bei dieser bevorzugten Ausführungsform gegeben, da hier eine Instrumentierung im Bereich zwischen 70 und zumindest 120° ermöglicht ist.

Im Querschnitt kann das gekrümmte Ablenkelement unterschiedliche Formen aufweisen und insbesondere als geschlossene bzw. teilweise offene Hülse bzw. rinnenförmig ausgebildet sein. Durch diese Ausbildung wird auch im Bereich des Ablenkelementes eine gute Führung erzielt.

Eine andere Ausgestaltung der Erfindung sieht vor, daß der Instrumentierkanal geschlossen bis an die seitliche Oberfläche des Endoskopes herangeführt ist. Angrenzend an den geschlossenen Bereich erstreckt sich dann das Ablenkelement, so daß übergangslos für eine gute Führung des Instrumentariums gesorgt ist.

Das Lager für das Ablenkelement kann als Drehlager ausgebildet sein. Mit Vorteil ist dieses Lager jedoch als Schiebelager ausgebildet, da bei einer derartigen Lagerung weniger Platz für die Ausfahrbewegung beansprucht wird.

Um Reibungskräfte beim Ausfahren des Ablenkelementes zu minimieren, ist das Ablenkelement und/oder das Schiebelager zweckmäßig mit einer Gleitbeschichtung versehen. Auch kommt eine Ausführung des Lagers als Wälzlager in Frage, wenn die Reibungskräfte bei der Betätigung des Ablenkelementes minimiert werden sollen. Im Krümmungsbereich des Instrumentierkanals sind die Krümmung und der Durchmesser des Instrumentierkanals zweckmäßig so aufeinander abgestimmt, daß das durch den Instrumentierkanal geführte Instrumentarium, je nach Anlage im Winkel zwischen 60 und 90° aus der seitlichen Endoskopoberfläche austritt.

In sehr zweckmäßiger Weise weist das gesamte Ablenkelement eine Krümmung von insbesondere einem Winkel von etwa 90° auf, so daß die durch den Instrumentierkanal geführte Instrumentierung mittels des Ablenkungselementes um etwa weitere 90° sicher ablenkbar ist.

Ein ausführungsbeispiel der Erfindung ist in der Zeichnung dargestellt und im folgenden beschrieben. Es zeigen:

Figur 1 einen Längsschnitt durch das Distalende eines Seitenblick-Endoskopes,

Figur 2 einen Längsschnitt durch das in Figur 1 dargestellte Seitenblick-Endoskop in einer Ebene senkrecht zur Schnittebene gemäß Figur 1,

Figur 3 eine schematische Darstellung des im absteigenden Schenkel eines Zwölffingerdarmes angeordneten Seitenblick-Endoskopes im Bereich der gemeinsamen Mündung von Gallengang und Pankreasgang,

Figur 4 einen Schnitt durch eine praktische Ausführung des Distalendes eines Seitenblick-Endoskopes, und

Figur 5 eine Seitenansicht des Distalendes des Seitenblick-Endoskopes gemäß Figur 4.

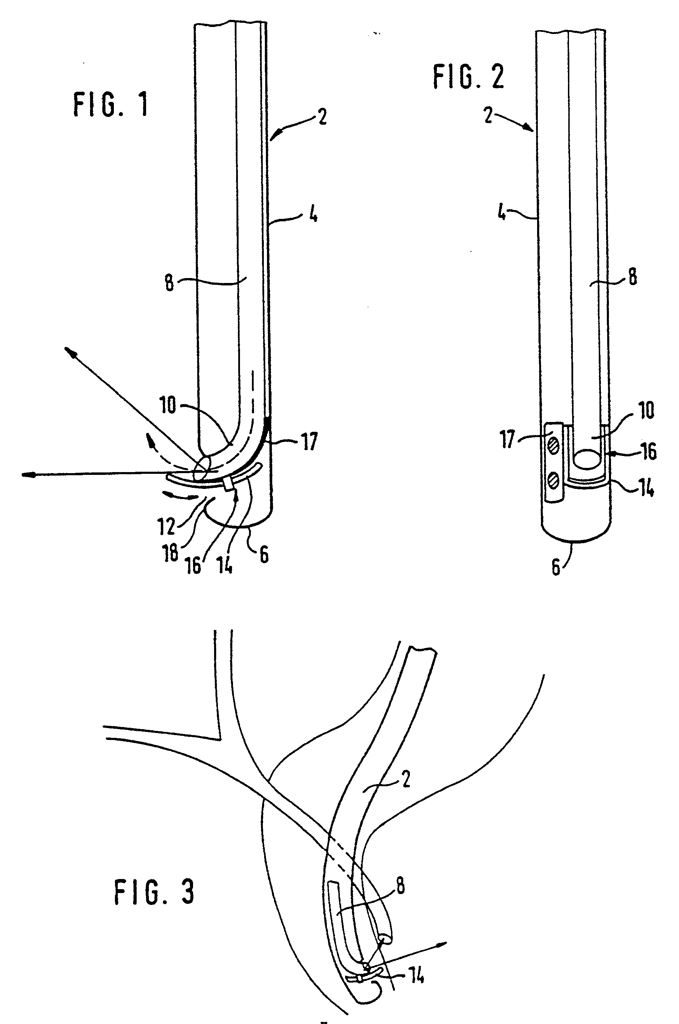

Es wird zunächst auf die Figuren 1 und 2 Bezug genommen, die Schnittdarstellungen des Distalendes, insbesondere des Arbeitsbereiches eines Seitenblick-Endoskopes 2 zeigen. Das Seitenblick-Endoskop 2 wird in diesem Bereich von einem flexiblen schlauchartigen Schaftteil bzw. Hüllkörper 4 gebildet, der am Ende einen Verschluß bzw. Boden 6 aufweist. Im Schaftteil 4, dessen anderes Ende mit einem (nicht dargestellten) Kopfteil verbunden ist, ist ein Instrumentierkanal 8 angeordnet, der sich praktisch über die ganze Länge des Schaftteiles 4 erstreckt, wobei die Achsen des Schaftteiles 4 und des Instrumentierkanals 8 im wesentlichen über die ganze Länge parallel zueinander verlaufen. Der Instrumentierkanal 8 ist hierbei zweckmäßig seitlich, in der Nähe der Schaftteilwandung angeordnet. Im Arbeitsbereich weist der Instrumentierkanal 8 einen gekrümmten Abschnitt 10 auf. Der gekrümmte Abschnitt 10 ist mit vorgegebener Krümmung geschlossen bis zur gegenüberliegenden Seitenwand des Schaftteiles bzw. Hüllkörpers 4 geführt und mündet dort nach außen. Der Schaftteil 4 weist zu diesem Zweck eine Öffnung 12 in seiner Wandung auf. Um eine Verschiebung des gekrümmten Abschnittes 10 zu vermeiden, ist der Abschnitt zweckmäßig an der Wand des Schaftteiles 4 befestigt bzw. abgestützt, wie bei 17 angedeutet ist.

Unterhalb des gekrümmten Abschnittes 10 ist ein Ablenkelement 14 gelagert. Das Lager für das Ablenkelement 14 ist hier als Schiebelager 16 ausgebildet. Es kann jedoch auch ein Schwenklager vorgesehen sein. Um ein Verschieben des Ablenkelementes 14 zu ermöglichen, sind seitlich am Instrumentierkanal 8 Lagerbacken vorgesehen, von denen eine mit 18 bezeichnet ist. Zwischen diesen Lagerbacken 18 ist das Ablenkelement 14 verschiebbar gelagert. Um die Verschiebebewegung mit möglichst geringem Kraftaufwand durchführen zu können, können die Lagerbacken 18 mit einem Kugel-, Rollen- oder Nadellager versehen sein. Alternativ kommt auch eine Gleitbeschichtung des Ablenkelementes und/oder der Lagerbacken 18 infrage. Am hinteren Ende des Ablenkelementes greift eine (nicht dargestellte) mechanische Betätigungseinrichtung an, mittels der das Ablenkelement vom Kopfteil des Endoskopes aus betätigbar ist. Das Ablenkelement 14 weist eine Krümmung sowohl in Längs- als auch in Querrichtung auf. Die Krümmung in Längsrichtung ist hierbei der Krümmung des Instrumentierkanals 8 weitgehend angepaßt. Bei einer Betätigung mittels der (nicht dargestellten) Betätigungseinrichtung wird das Ablenkelement 14 aus seiner (in auszogenen Linien dargestellten) Ruhestellung in eine, in gestrichelten Linien dargestellte ausgefahrene Stellung bewegt. Da das Lager, 16 im Bereich der Mündung des Instrumentierkanals bzw. im Bereich der Öffnung 12 angeordnet ist, erstreckt sich in der ausgefahrenen Stellung praktisch das ganze Ablenkelement 14 außerhalb des Hüllkörpers. Zusätzlich zu der durch die Krümmung des Instrumentierkanals 8 bereits erreichten Abbiegung kann hiermit ein weiterer Abbiegungsbereich von, je nachdem wie weit der Ablenkelement 14 ausgefahren ist, etwa bis zu 90° erzielt werden. Bei einem um 90° ausgefahrenen Ablenkelement sind hierbei besondere Maßnahmen erforderlich, um dem Ablenkelement in dieser Stellung eine hinreichend bestimmte Führung zu geben. Diese zusätzlichen Maßnahmen können beispielsweise in einer teleskopartigen Gestaltung des Ablenkelementes bestehen.

Der Instrumentierkanal besteht zweckmäßig aus besonders gleitfähigen und stabilen Materialien. Infrage kommen hier insbesondere Keramik bzw. Kunststoff.

Das Gleitmaterial kann auch als Beschichtung aufgebracht sein. Entsprechendes gilt für das Ablenkelement 14.

Im Folgenden ist das technische Vorgehen bei der Einbringung eines Verweilkatheters mittels des erfindungsgemäßen Seitenblick-Endoskopes beschrieben. Hierbei wird zunächst das Seitenblick-Endoskop mit seinem Distalende in der Mitte des absteigenden Schenkels des Zwölffingerdarmes plaziert. Zunächst wird eine kleine Papillotomie (Schlitzung des Ausführungsganges der Gallenwege) durchgeführt. Nach anschließender Einbringung eines dünnen Führungsdrahtes (Seldinger Drahtes) in den Gallengang, der unter röntgenologischer Sicht bis in die intrahepatischen Gallenwege vorgeschoben wird, wird der Verweilkatheter über den liegenden Führungsdraht in den Instrumentierkanal des Endoskopes vorgeschoben und anschließend mit einem nachgeschobenen Schubkatheter mit Druck durch die Gallenwegsverengung hindurch so plaziert, daß eine.Ableitung gewährleistet wird. Anschließend werden der Führungsdraht sowie der Schubkatheter durch den Instrumentierkanal entfernt.

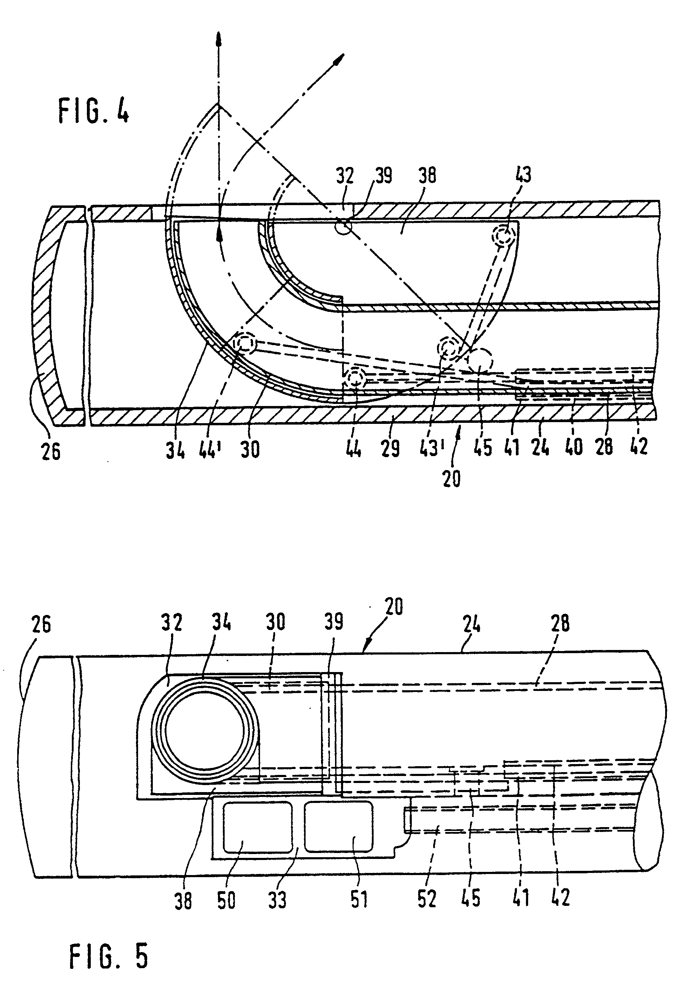

Die Figuren 4 und 5 zeigen eine praktische Ausführung eines Distalendes eines Seitenblick-Endoskopes 20. Das Seitenblick-Endoskop 20 wird in diesem Bereich von einem flexiblen schlauchartigen Schaftteil 24 gebildet, der am Ende einen Boden 26 bildet. Zum Schaftteil 24 ist ein Instrumentierkanal 28 angeordnet, der im wesentlichen parallel zur Seitenwand 29 des Schaftteiles 24 verläuft und dicht neben dieser angeordnet ist. Im unteren Bereich weist der Instrumentierkanal 28 einen gekrümmten Abschnitt 30 auf. Dieser schließt einen Winkel von im wesentlichen 90° ein und führt zu einer seitlichen Öffnung 32 in der Wand des Schaftteiles 24. Auf dem gekrümmten Abschnitt 30 sitzt ein Ablenkelement 34, das ein geschlossenes Rohrstück aufweist, welches von einem Dreh-Lagerelement 38 gehalten ist. Das Dreh-Lagerelement 38 ist auf einer Drehachse 39 gelagert, die dicht neben der Wand des Schaftteiles 24 und nahe der Begrenzung der Öffnung 32 angeordnet ist.

Zur Betätigung des Ablenkelementes 34 ist ein Zugdrahtmechanismus vorgesehen, der zum Kopf des Endoskopes führt. Hierzu ist parallel zum Schaftteil 24 ein Rohr bzw. Kanal 40 vorgesehen, in dem Zugdrähte 41, 42 geführt sind, die zu am Dreh-Lagerelement 28 angeordneten Befestigungspunkten 43, 44 geführt und dort befestigt sind. Die Zugdrähte sind hierbei um eine Umlenkrolle 45 geführt, die im Endbereich des Instrumentierkanals 28 angeordnet und am Schaftteil 24 gelagert ist. Ein erster Zugdraht 41 führt zu einem ersten Befestigungspunkt 43, der vorgesehen ist, um das Dreh-Lagerelement 38 und damit s Ablenkelement 34 aus der Ausgangsstellung (in F: 4 in ausgezogenen Linien dargestellt in einer aus fahrenen Stellung) 43' zu bewegen, in der das Dreh-Lagerelement 38 mit dem Ablenkelement 34 um z.B. 45° ausgefahren ist und dadurch eine Ablenkung von 135° ermöglicht. Der andere Zugdraht 42 ist am Befestigungsdpunkt 44 angebracht. Dieser Zugdraht dient dazu, das Dreh-Lagerelement 28 und das Ablenkelement 34 wieder in die Ausgangsstellung zurückzuführen. Wie weiter oben bezüglich der Ausführung gemäß Figuren 1 und 2 bereits angegeben, sind das Ablenkelement 34 und der gekrümmte Abschnitt 30 des Instrumentierkanals zweckmäßig mit einer Gleichbeschichtung oder mit speziellen Wälzlagern versehen, um eine leichte Betätigung sicherzustellen.

Figur 5 zeigt eine Seitenansicht des Seitenblick-Endoskopes 20. Aus dieser Ansicht ist ersichtlich, daß die Öffnung 32 einen etwa rechteckigen Querschnitt aufweist und daß angrenzend an die Öffnung 32 eine weitere Öffnung 33 vorgesehen ist, in der eine Lichtquelle 50 und ein Objektiv 51 angeordnet sind. In dieser Öffnung 33 mündet auch ein Wasserkanal 52, der parallel zum Instrumentierkanal verlaufend im Schaftteil 24 angeordnet ist.

## Patentansprüche

1. Seitenblick-Endoskop mit einem Instrumentierkanal (8), der in Arbeitsbereich (Distalende) eine Austrittsöffnung bildet, und mit einem Ablenkelement (14) und einer Betätigungseinrichtung für dieses, wobei der Instrumentierkanal (8) am Austrittsende einen gekrümmten Abschnitt (10) aufweist, der im Arbeitsbereich zu der in der seitlichen Wandung des Endoskopes vorgesehenen Austrittsöffnung (12) hin gerichtet ist, dadurch gekennzeichnet, daß im gekrümmten Abschnitt des Instrumentierkanals (8) die Krümmung und der Durchmesser des Instrumentierkanals (8) so aufeinander abgestimmt sind, daß das durch den Instrumentierkanal geführte Instrumentarium je nach Ausbildung in einem vorgegebenen Ablenkwinkel zwischen 60 und 90° aus der seitlichen Wandung des Hüllkörper (4) austritt, und daß das Ablenkelement (14) gekrümmt ist und einen Winkel sektor von etwa 60 bis 90° einschließt, so daß das durch den Instrumentierkanal geführte Instrumentarium mittels des Ablenkelementes (14) bis 90° zusätzlich zu dem vorgegebenen Ablenkwinkel ablenkbar ist.

2. Seitenblick-Endoskop nach Anspruch 1, dadurch gekennzeichnet, daß der Instrumentierkanal (8), insbesondere geschlossen, bis an die seitliche Austrittsöffnung des Endoskopes geführt ist.

3. Seitenblick-Endoskop nach einem der vorhergehenden Ansprüche, bei dem die Betätigungseinrichtung mechanische Mittel aufweist, die von einem Kopfteil des Endoskopes zum Ablenkelement (14) führen, dadurch gekennzeichnet, daß die mechanischen Mittel Zugdrähte (46,47) aufweisen, die insbesondere um zumindest eine Umlenkwelle (45) geführt sind.

4. Seitenblick-Endoskop nach einem der vorhergehenden Ansprüche, dadurch gekennzeichnet, daß das Ablenkelement (14) in Querrichtung gekrümmt ist.

5. Seitenblick-Endoskop nach einem der vorhergehenden Ansprüche, dadurch gekennzeichnet, daß das Ablenkelement (14) als ganz oder teilweise geschlossene Hülse bzw. Rohr ausgeführt ist.

6. Seitenblick-Endoskop nach einem der vorhergehenden Ansprüche, bei dem das Larger für das Ablenkelement als Drehlager ausgebildet ist, dadurch gekennzeichnet, daß das Ablenkelement (34) an einem Dreh-Larger-element (28) gehalten ist.

7. Seitenblick-Endoskop nach einem der Ansprüche 1 bis 5, dadurch gekennzeichnet, daß das Larger für das Ablenkelement (14) als Schiebelager (16) ausgebildet ist.

8. Seitenblick-Endoskop nach Anspruch 7, dadurch gekennzeichnet, daß das Ablenkelement (14) und/oder das Schiebelager (16) eine Gleitbeschichtung aufweisen.

9.Seitenblick-Endoskop nach Anspruch 7, dadurch gekennzeichnet, daß das Schiebelager (16) als Wälzlager ausgebildet ist.

## Claims

1. Side-viewing endoscope with an instrument duct (8) which forms an outlet opening in the operating part (distal end), and with a deflector element (14) and an operating device therefor, the instrument duct (8) having at the outlet end a curved section (10) which in the operating part points towards the outlet opening (12) in the side wall of the endoscope, characterized in that the curvature and diameter of the instrument duct (8) in the curved section of the instrument duct (8) are correlated with one another so that the instrument passed through the instrument duct emerges from the side wall of the sheath (4) with a predetermined angle of deflection of between 60 and 90° according to its design, and in that the deflector element (14) is curved and includes an angle sector of approximately 60 to 90°, so that the instrument passed through the instrument duct can be deflected by the deflector element (14) by up to 90° in addition to the predetermined angle of deflection.

2. Side-viewing endoscope according to Claim 1, characterized in that the instrument duct (8), in particular closed, is guided to the lateral outlet opening of the endoscope.

3. Side-viewing endoscope according to one of the preceding claims, in which the operating device has mechanical means which run from a head part of the endoscope to the deflector element (14), characterized in that the mechanical means have pull wires (46, 47) which in particular are passed around at least one deflector shaft (45).

4. Side-viewing endoscope according to one of the preceding claims, characterized in that the deflector element (14) is curved in the transverse direction.

5. Side-viewing endoscope according to one of the preceding claims, characterized in that the deflector element (14) is constructed as a wholly or partially closed sleeve or tube.

6. Side-viewing endoscope according to one of the preceding claims, in which the bearing for the deflector element is constructed as a pivotal bearing, characterized in thai the deflector element (34) is mounted on a pivotal bearing element (28).

7. Side-viewing endoscope according to one of claims 1 to 5, characterized in that the bearing for the deflector element (14) is constructed as a sliding bearing (16).

8. Side-viewing endoscope according to Claim 7, characterized in that the deflector element (14) and/or the sliding bearing (16) are lined with a low-friction material.

9. Side-viewing endoscope according to Claim 7, characterized in that the sliding bearing (16) is constructed as a rolling bearing.

## Revendications

1. Endoscope pour recherche latérale comprenant un canal d'instrumentation (8) qui, dans la zone de travail (extrémité distale), forme un orifice de sortie et comporte un élément déflecteur (14) et un dispositif d'actionnement pour ce dernier, le canal d'instrumentation (8) comportant à son extrémité de sortie une partie incurvée (10) qui, dans la zone de travail, est dirigée vers l'orifice de sortie (12) prévu dans la paroi latérale de l'endoscope, caractérisé en ce que, dans la partie incurvée du canal d'instrumentation (8), la courbure et le diamètre du canal d'instrumentation (8) sont adaptés l'un à l'autre de telle manière que l'ensemble-instrument guidé à travers le canal d'instrumentation sort de la paroi latérale du corps enveloppant (4) selon un angle de déviation prédéterminé compris, en fonction de la structure, entre 60 et 90° et en ce que l'élément déflecteur (14) est incurvé et comprend un secteur angulaire d'environ 60 à 90° de sorte que l'instrumentation guidée par le canal d'instrumentation peut, au moyen de l'élément déflecteur (14), être dévié, jusqu'à 90°, au-delà de l'angle de déviation prédéterminé.

2. Endoscope pour recherche latérale selon la revendication 1, caractérisé en ce que le canal d'instrumentation (8), en particulier fermé, est guidé jusqu'à l'orifice de sortie latérale de l'endoscope.

3. Endoscope pour recherche latérale selon l'une des revendications précédentes dans lequel le dispositif d'actionnement comporte des moyens mécaniques qui conduisent depuis une partie de tête de l'endoscope jusqu'à l'élément déflecteur (14), caractérisé en ce que les moyens mécaniques comportent des fils de traction (46, 47) qui sont en particulier guidés au moins autour d'un arbre de renvoi (45).

4. Endoscope pour recherche latérale selon l'une des revendications précédentes, caractérisé en ce que l'élément déflecteur (14) est incurvé en direction transversale.

5. Endoscope pour recherche latérale selon l'une des revendications précédentes, caractérisé en ce que l'élément déflecteur (14) est réalisé sous forme de manchon ou de tube totalement ou partiellement fermé.

6. Endoscope pour recherche latérale selon l'une des revendications précédentes, dans lequel le logement de l'élément déflecteur est réalisé sous forme de palier rotatif, caractérisé en ce que l'élément de déflecteur (34) est maintenu sur un élément (28) de palier rotatif.

7. Endoscope pour recherche latérale selon l'une des revendications 1 à 5, caractérisé en ce que le logement de l'élément déflecteur (14) est réalisé sous forme de logement coulissant (16).

8. Endoscope pour recherche latérale selon la revendication 7, caractérisé en ce que l'élément déflecteur (14) et/ou le logement à coulissant (16) présente un revêtement de glissement.

9. Endoscope pour recherche latérale selon la revendication 7, caractérisé en ce que le logement coulissant (16) est réalisé sous forme de logement à rouleaux.

EP 0 271 545 B1

FIG. 1

FIG. 2

FIG. 3

# FIG. 4

# FIG. 5